# EUROPEAN PATENT SPECIFICATION

(11) **EP 1 096 924 B1**
(45) Date of publication and mention of the grant of the patent: **22.11.2006**
(21) Application number: 99933827.0
(22) Date of filing: 09.07.1999
(51) Int. Cl.: A61K 31/00, A61Q 7/00, A61P 17/14

(54) **INHIBITORS OF PROTEASOMAL ACTIVITY FOR STIMULATING HAIR GROWTH**
INHIBITOREN DER PROTEASOMALEN AKTIVITÄT ZUR STIMULIERUNG DES HAARWUCHSES
INHIBITEURS D'ACTIVITE PROTEASOMIQUE POUR LA STIMULATION DE LA CROISSANCE CAPILLAIRE

(30) Priority: 10.07.1998 US 113947
(43) Date of publication of application: 09.05.2001
(73) Proprietor: Osteoscreen, Inc., San Antonio, TX 78229 (US)
(72) Inventor: MUNDY, Gregory, R., San Antonio, TX 78230 (US); GARRETT, I., Ross, San Antonio, TX 78023 (US); ROSSINI, G., San Antonio, TX 78238 (US)
(74) Representative: Sexton, Jane Helen
(86) International application number: PCT/US1999/015533
(87) International publication number: WO 2000/002548

(56) References cited:
- EP-A- 0 931 544
- WO-A-97/09315
- WO-A-97/23457
- WO-A-97/38699
- WO-A-98/25460
- DE-A- 19 716 713
- US-A- 5 580 854
- PATENT ABSTRACTS OF JAPAN vol. 17, no. 436 (C-1096), 12 August 1993 (1993-08-12) & JP 05 097697 A (LION CORP), 20 April 1993 (1993-04-20)
- K. OZAKI ETAL: "NF-kB inhibitors stimulate apoptosis of rabbit mature osteoclasts and inhibit bone resorption by these cells" FEBS LETTERS, vol. 410, 1997, pages 297-300, XP002124509
- G. LUTZ: "Effects of Cyclosporin A on Hair" SKIN PHARMACOLOGY, vol. 7, January 1994 (1994-01) - April 1994 (1994-04), pages 101-104, XP002124510
- A. K. GUPTA ET AL: "Oral cyclosporine for the treatment of alopecia areata" JOURNAL OF THE AMERICAN ACADEMY OF DERMATOLOGY, vol. 22, no. 2, February 1990 (1990-02), pages 242-250, XP002124511
- PATENT ABSTRACTS OF JAPAN vol. 11, no. 193 (C-430), 20 June 1987 (1987-06-20) & JP 62 019513 A (SHISEIDO CO LTD), 28 January 1987 (1987-01-28)
- PATENT ABSTRACTS OF JAPAN vol. 10, no. 112 (C-342), 25 April 1986 (1986-04-25) & JP 60 243008 A (WAKAMOTO SEIYAKU KK), 3 December 1985 (1985-12-03)
- ADAMS J ET AL: "CHAPTER 28. NOVEL INHIBITORS OF THE PROTEASOME AND THEIR THERAPEUTIC USE IN INFLAMMATION" ANNUAL REPORTS IN MEDICINAL CHEMISTRY,US,SAN DIEGO,1996, page 279-288 XP000791150 ISSN: 0065-7743
- VINITSKY A ET AL: "INHIBITION OF THE PROTEOLYTIC ACTIVITY OF THE MULTICATALYTIC PROTEINASE COMPLEX (PROTEASOME) BY SUBSTRATE-RELATED PEPTIDYL ALDEHYDES" JOURNAL OF BIOLOGICAL CHEMISTRY,US,AMERICAN SOCIETY OF BIOLOGICAL CHEMISTS, BALTIMORE, MD, vol. 269, no. 47, 1994, page 29860-29866 XP002039864 ISSN: 0021-9258

## Description

### Technical Field

The invention relates to compositions and methods for use in enhancing hair density and growth. More specifically, the invention concerns the use of inhibitors of proteasomal activity for this purpose.

### Background Art

Inhibitors of proteasomal activity, and to some extent inhibitors of NF-κB activity, have two important physiological effects. First, they are able to enhance bone formation and are thus useful for treating various bone disorders. Second, they stimulate the production of hair follicles and are thus useful in stimulating hair growth, including hair density, in subject where this is desirable.

### Effect on Bone

Bone is subject to constant breakdown and resynthesis in a complex process mediated by osteoblasts, which produce new bone, and osteoclasts, which destroy bone. The activities of these cells are regulated by a large number of cytokines and growth factors, many of which have now been identified and cloned.

There is a plethora of conditions which are characterized by the need to enhance bone formation or to inhibit bone resorption. Perhaps the most obvious is the case of bone fractures, where it would be desirable to stimulate bone growth and to hasten and complete bone repair. Agents that enhance bone formation would also be useful in facial reconstruction procedures. Other bone deficit conditions include bone segmental defects, periodontal disease, metastatic bone disease, osteolytic bone disease and conditions where connective tissue repair would be beneficial, such as healing or regeneration of cartilage defects or injury. Also of great significance is the chronic condition of osteoporosis, including age-related osteoporosis and osteoporosis associated with post-menopausal hormone status. Other conditions characterized by the need for bone growth include primary and secondary hyperparathyroidism, disuse osteoporosis, diabetes-related osteoporosis, and glucocorticoid-related osteoporosis.

There are currently no satisfactory pharmaceutical approaches to managing any of these conditions. Bone fractures are still treated exclusively using casts, braces, anchoring devices and other strictly mechanical means. Further bone deterioration associated with post-menopausal osteoporosis has been treated with estrogens or bisphosphonates, which may have drawbacks for some individuals. Although various approaches have been tried, as further discussed below, there remains a need for additions to the repertoire of agents which can be used to treat these conditions.

Treatment of bone or other skeletal disorders, such as those associated with cartilage, can be achieved either by enhancing bone formation or inhibiting bone resorption or both. A number of approaches have been suggested which relate to bone formation.

Bone tissue is an excellent source for factors which have the capacity for stimulating bone cells. Thus, extracts of bovine bone tissue obtained from slaughterhouses contain not only structural proteins which are responsible for maintaining the structural integrity of bone, but also biologically active bone growth factors which can stimulate bone cells to proliferate. Among these latter factors are transforming growth factor β, the heparin-binding growth factors (e.g., acidic and basic fibroblast growth factor), the insulin-like growth factors (e.g., insulin-like growth factor I and insulin-like growth factor II), and a recently described family of proteins called bone morphogenetic proteins (BMPs). All of these growth factors have effects on other types of cells, as well as on bone cells.

The BMPs are novel factors in the extended transforming growth factor β superfamily. Recombinant BMP2 and BMP4 can induce new bone formation when they are injected locally into the subcutaneous tissues of rats (Wozney *J Molec Reprod Dev* (1992) 32:160-67). These factors are expressed by normal osteoblasts as they differentiate, and have been shown to stimulate osteoblast differentiation and bone nodule formation *in vitro* as well as bone formation *in vivo* (Harris S *et al. J Bone Miner Res* (1994) 9:855-63). This latter property suggests potential usefulness as therapeutic agents in diseases which result in bone loss.

The cells which are responsible for forming bone are osteoblasts. As osteoblasts differentiate from precursors to mature bone-forming cells, they express and secrete a number of enzymes and structural proteins of the bone matrix, including Type-1 collagen, osteocalcin, osteopontin and alkaline phosphatase. They also synthesize a number of growth regulatory peptides which are stored in the bone matrix, and are presumably responsible for normal bone formation. These growth regulatory peptides include the BMPs (Harris S. *et al.* (1994), *supra).* In studies of primary cultures of fetal rat calvarial osteoblasts, BMPs 1, 2, 3, 4, and 6 are expressed by cultured cells prior to the formation of mineralized bone nodules (Harris S*. et al.* (1994), *supra*). Like alkaline phosphatase, osteocalcin and osteopontin, the BMPs are expressed by cultured osteoblasts as they proliferate and differentiate.

Although the BMPs are potent stimulators of bone formation *in vitro* and *in vivo,* there are disadvantages to their use as therapeutic agents to enhance bone healing. Receptors for the bone morphogenetic proteins have been identified in many tissues, and the BMPs themselves are expressed in a large variety of tissues in specific temporal and spatial patterns. This suggests that BMPs may have effects on many tissues in addition to bone, potentially limiting their usefulness as therapeutic agents when administered systemically. Moreover, since they are peptides, they would have to be administered by injection. These disadvantages impose severe limitations to the development of BMPs as therapeutic agents.

The fluorides, suggested also for this purpose, have a mode of action which may be related to tyrosine phosphorylation of growth factor receptors on osteoblasts, as described, for example, Burgener *et al. J Bone Min Res* (1995) 10:164-171, but administration of fluorides is associated with increased bone fragility, presumably due to effects on bone mineralization.

Small molecules which are able to stimulate bone formation have been disclosed in PCT applications WO98/17267 published 30 April 1998, WO97/15308 published 1 May 1997 and WO97/48694 published 24 December 1997. These agents generally comprise two aromatic systems spatially separated by a linker. In addition, PCT application WO98/25460 published 18 June 1998 discloses the use of the class of compounds known as statins in enhancing bone formation. U.S. application Serial No. 09/096,631 filed 12 June 1998 is directed to compounds for stimulating bone growth that are generally isoprenoid pathway inhibitors. The contents of this application, as well as that of the PCT applications cited above, are incorporated herein by reference.

Other agents appear to operate by preventing the resorption of bone. Thus, U.S. Patent No. 5,280,040 discloses compounds described as useful in the treatment of osteoporosis. These compounds putatively achieve this result by preventing bone resorption.

Wang, G.-J. *et al., J Formos Med Assoc* (1995) 94:589-592 report that certain lipid clearing agents, exemplified by lovastatin and bezafibrate, were able to inhibit the bone resorption resulting from steroid administration in rabbits. There was no effect on bone formation by these two compounds in the absence of steroid treatment. The mechanism of the inhibition in bone resorption observed in the presence of steroids (and the mechanism of the effect of steroid on bone *per se)* is said to be unknown.

An abstract entitled "Lovastatin Prevents Steroid-Induced Adipogenesis and Osteoporosis" by Cui, Q. *et al.* appeared in the Reports of the ASBMR 18th Annual Meeting (September 1996) *J Bone Mineral Res.* (1996) 11(S1):S510 which reports that lovastatin diminished triglyceride vesicles that accumulated when osteoprogenitor cells cloned from bone marrow stroma of chickens were treated in culture with dexamethasone. Lovastatin was reported to diminish the expression of certain mRNAs and to allow the cells to maintain the osteogenic phenotype after dexamethasone treatment, and chickens that had undergone bone loss in the femoral head as a result of dexamethasone treatment were improved by treatment with lovastatin.

These data are, however, contrary to reports that dexamethasone and other inducers, such as BMPs, induce osteoblastic differentiation and stimulate osteocalcin mRNA (Bellows, *C.G., et al., Develop Biol* (1990) 140:132-38; Rickard, D.J., *et al., Develop Biol* (1994) 161:218-28). In addition, Ducy, P. *et al., Nature* (1996) 382:448-52 have recently reported that osteocalcin deficient mice exhibit a phenotype marked by increased bone formation and bones of improved functional quality, without impairment of bone resorption. Ducy *et al.* state that their data suggest that osteocalcin antagonists may be of therapeutic use in conjunction with estrogen replacement therapy (for prevention or treatment of osteoporosis).

It has also been shown that lovastatin inhibits lipopolysaccharide-induced NF-κB activation in human mesangial cells. Guijaro, C. *et al. Nephrol Dial Transplant* (1996) 11:6:990-996.

### And Its Effect on Hair Growth

Disorders of human hair growth include male pattern baldness, alopecia areota, alopecia induced by cancer chemotherapy and hair thinning associated with aging. These conditions are poorly understood, but nevertheless common and distressing, since hair is an important factor in human social and sexual communication.

Hair follicle regulation and growth are still not well understood, but represent dynamic processes involving proliferation, differentiation and cellular interactions during tissue morphogenesis. It is believed that hair follicles are formed only in early stages of development and not replaced.

Hardy, M.H. *et al. Trans Genet* (1992) 8:55-61 describes evidence that bone morphogenetic proteins (BMPs), members of the TGFβ super family, are differentially expressed in hair follicles during development. Harris, S.E. *et al. J Bone Miner Res* (1994) 9:855-863 describes the effects of TGFβ on expression of BMP-2 and other substances in bone cells. BMP-2 expression in mature follicles also occurs during maturation and after the period of cell proliferation (Hardy *et al.* (1992, *supra).* As noted, however, by Blessing, M. *et al. Genes and Develop* (1992) 7:204-215, the precise role functional role of BMP-2 in hair follicle maturation remains unclear.

Approaches to treat baldness abound in the U.S. patent literature. See for example U.S. Patent No. 5,767,152 (cyanocarboxylic acid derivatives), U.S. Patent No. 5,824,643 (keratinocyte growth factors) and U.S. Patent No. 5,910,497 (16-pyrazinyl-substitute-4-aza-androstane 5-alpha.-reductase isozyme 1 inhibitors). There are many others.

Gat, U. *et al. Cell* (1998) 95:605-614 has demonstrated that β-catenin causes adult epithelial cells to create hair follicles, a surprising result in light of the known inability of mature cells to do so. B-Catenin is known to play a role in cell-cell adhesion and growth factor signal transfection. It is also known that after ubiquitination, β-catenin is degraded by the proteasomes. Orford, K *et al*. *J Chem* (1997) 272:24735-24738. At least one gene associated with hair growth (or lack thereof) has also been reported. Ahmed, W*. et al. Science* (1998) 279:720-724.

Two accepted agents currently used for the treatment of hair loss are the antihypertensive drug Minoxidil and the 5α-reductase inhibitor Finasteride. Neither is entirely satisfactory. Both suffer from modest efficacy and are inconvenient to administer. A specific, topically active and easy to administer compound with better efficacy than these agents would represent a marked advance.

### Proteasomes and NF-κB

The present invention discloses convenient assays for compounds that will be useful in stimulating hair growth. The assays involve inhibition of the activity of proteasomal proteases, preferably proteasomal proteases. Compounds which inhibit this activity are generally useful in treating hair growth disorders. Compounds that inhibit the production of these proteases will also be useful in the invention. Their ability to do so can be further confirmed by additional assays.

The proteasome is a noncompartmentalized collection of unrelated proteases which form a common architecture in which proteolytic subunits are self-assembled to form barrel-shaped complexes (for review, see Baumeister *et al., Cell* (1998) 92:367-380. The proteasome contains an array of distinct proteolytic activities inside eukaryotic cells. Compounds which inhibit proteasomal activity also reduce NF-κB activity by limiting its capacity to be translocated to the nucleus (Barnes, P.J. *et al. New Engl J Med* (1997) 336:1066-1071.

### Disclosure of the Invention

The present invention adds to the repertoire of hair growth stimulating agents by providing drugs which would inhibit key proteins and enzymes involved in proteasomal activity and thus stimulate hair growth. In accordance with the present invention, we have discovered that inhibition of the functions of the proteasomal proteins in bone cells leads to hair follicle formation and stimulation. Thus, assessing a candidate compound for its ability to inhibit proteasomal proteins provides a useful means to identify hair growth anabolic agents.

The present specification thus provides methods for identification of compounds that stimulate hair growth by assessing their capacity to inhibit proteasome activity. Also useful in the methods of the invention are compounds which inhibit the *in situ* production of the enzymes contained in the proteasome. Once a compound found to inhibit these activities has been identified, it can be used in an additional aspect of the invention -- a method to stimulate the growth of hair by contacting suitable cells with the identified compound. The cellular contact may include *in vivo* administration and the compounds of the invention are thus useful in treating baldness, alopecia and the like. These methods are performed, according to the present invention, with compounds identified as inhibitors of proteasome activity or inhibitors of the production of the proteasome enzymes.

In a first aspect the invention refers to a cosmetic method to treat a mammalian subject for a condition benefited by stimulating hair growth which method comprises administering to said mammalian subject an amount effective to stimulate hair growth of a compound that inhibits proteasomal activity or that inhibits production of these proteins, wherein said compound is a statin, a peptidyl, aldehyde or a peptidyl epoxyketone, or is selected from the group consisting of pentoxyfilline, gliotoxin, SN50, Bay 11-7082, capsaicin, PDTC, PPM-18, lactacystin and MG262.

In a further aspect the invention relates to the use of a compound that inhibits proteasomal activity or that inhibits production of these proteins in the manufacture of a medicament for stimulating hair growth in a mammalian subject wherein said compound is a statin, a peptidyl aldehyde, or a peptidyl epoxyketone, or is selected from the group consisting of pentoxyfilline, gliotoxin, SN50, Bay 11-7082, capsaicin, PDTC, PPM-18, lactacystin and MG262.

### Brief Description of the Drawings

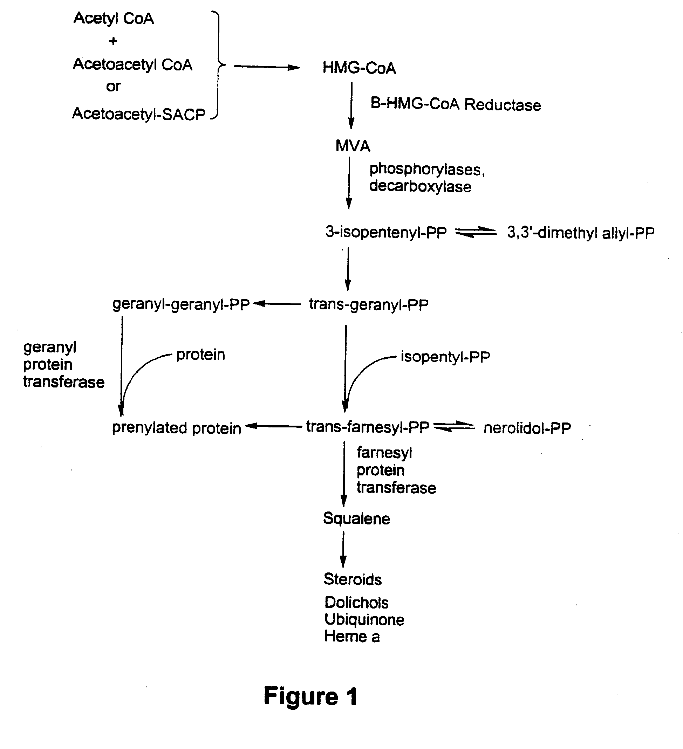

Figure 1 shows a diagram of the isoprenoid pathway.

### Modes of Carrying Out the Invention

In accordance with the present invention, there are provided methods of treating disorders of hair growth. Disorders of hair growth may be the result of a defect in the ability of existing hair follicles to extrude hair, or may be the result of a deficiency in the number of hair follicles *per se.* "Stimulation of hair growth" refers to increasing the volume of hair in a particular area of a subject whether this is the result of an increased rate of growth in length and/or thickness from the same number of hair follicles, growth proceeding from an enhanced number of hair follicles, or both. The number of hair follicles can be enhanced by further activating existing hair follicles or by stimulating the appearance or proliferation of hair follicles in a particular region of the skin.

As employed herein, the term "subject" embraces human as well as other animal species, such as, for example, canine, feline, bovine, porcine, rodent, and the like. It will be understood by the skilled practitioner that the subject is one appropriate to the desirability of stimulating hair growth. Thus, in general, for example, stimulation of hair growth will be confined in most instances to animals that would appropriately exhibit such growth.

Conditions which would be benefited by "treating" or "treatment" for stimulation of hair growth include male pattern baldness, alopecia caused by chemotherapy, hair thinning resulting from aging, genetic disorders which result in deficiency of hair coverage, and, in animals, providing additional protection from cold temperatures. Thus, while use in humans may be primarily of cosmetic benefit, use in animals may be therapeutic as well.

The compositions of the invention may be administered systemically or locally. For systemic use, the compounds herein are formulated for parenteral (e.g., intravenous, subcutaneous, intramuscular, intraperitoneal, intranasal or transdermal) or enteral (e.g., oral or rectal) delivery according to conventional methods. Intravenous administration can be by a series of injections or by continuous infusion over an extended period. Administration by injection or other routes of discretely spaced administration can be performed at intervals ranging from weekly to once to three times daily. Alternatively, the compounds disclosed herein may be administered in a cyclical manner (administration of disclosed compound; followed by no administration; followed by administration of disclosed compound, and the like). Treatment will continue until the desired outcome is achieved. In general, pharmaceutical formulations will include a compound of the present invention in combination with a pharmaceutically acceptable vehicle, such as saline, buffered saline, 5% dextrose in water, borate-buffered saline containing trace metals or the like. Formulations may further include one or more excipients, preservatives, solubilizers, buffering agents, albumin to prevent protein loss on vial surfaces, lubricants, fillers, stabilizers, etc. Methods of formulation are well known in the art and are disclosed, for example, in Remington's Pharmaceutical Sciences, latest edition, Mack Publishing Co., Easton PA, which is incorporated herein by reference. Pharmaceutical compositions for use within the present invention can be in the form of sterile, non-pyrogenic liquid solutions or suspensions, coated capsules, suppositories, lyophilized powders, transdermal patches or other forms known in the art. Local administration may be by injection at the site of injury or defect, or by insertion or attachment of a solid carrier at the site, or by direct, topical application of a viscous liquid, or the like. For local administration, the delivery vehicle preferably is a vehicle that can be absorbed by the subject without adverse effects.

In additional formulations, conventional preparations such as those described below may be used.

Aqueous suspensions may contain the active ingredient in admixture with pharmacologically acceptable excipients, comprising suspending agents, such as methyl cellulose; and wetting agents, such as lecithin, lysolecithin or long-chain fatty alcohols. The said aqueous suspensions may also contain preservatives, coloring agents, flavoring agents, sweetening agents and the like in accordance with industry standards.

Preparations for topical and local application comprise aerosol sprays, lotions, gels and ointments in pharmaceutically appropriate vehicles which may comprise lower aliphatic alcohols, polyglycols such as glycerol, polyethylene glycol, esters of fatty acids, oils and fats, and silicones. The preparations may further comprise antioxidants, such as ascorbic acid or tocopherol, and preservatives, such as p-hydroxybenzoic acid esters.

Parenteral preparations comprise particularly sterile or sterilized products. Injectable compositions may be provided containing the active compound and any of the well known injectable carriers. These may contain salts for regulating the osmotic pressure.

Veterinary uses of the disclosed compounds are also contemplated, as set forth above. Such uses would include treatment of defects associated with hair or fur in domestic animals, livestock and thoroughbred horses.

As stated above, the compounds of the present invention may also be used to stimulate the growth of hair either by enhancing its rate of formation from existing follicles, stimulating inactive follicles, effecting the production of additional hair follicles or some combination of the foregoing, or by any other mechanism that may or may not presently be understood.

Within the present invention, an "effective amount" of a composition for use in stimulating hair growth is that amount which provides the desired effect in terms of length or density of hair. Such effective amounts will be determined using routine optimization techniques and are dependent on the particular condition to be treated, the condition of the patient, the route of administration, the formulation, and the judgment of the practitioner and other factors evident to those skilled in the art. The dosage required for the compounds of the invention is manifested as differences between successfully treated subjects and controls with regard to stimulation of hair growth and can generally be ascertained by direct observation.

The dosage of the compounds of the invention will vary according to the extent and severity of the need for treatment, the activity of the administered compound, the general health of the subject, and other considerations well known to the skilled artisan. Generally, they can be administered to a typical human on a daily basis as an oral dose of about 0.1 mg/kg-1000 mg/kg, and more preferably from about 1 mg/kg to about 200 mg/kg. The parenteral dose will appropriately be 20-100% of the oral dose. While topical administration is generally preferable for stimulating hair growth, as generally only local effects are desired, systemic treatment may be preferable in some instances as well.

### Assays for Compounds Useful in the Invention

Assays for assessing the ability of a compound to inhibit proteasomal activity and for inhibitors of NF-κB activity are well known in the art. Two typical, but nonlimiting assays are described below.

### Assessment of Proteasomal Activity

Proteasomal activity is measured by an increase in cytoplasmic ubiquitinylated protein complexes, as assessed by Western blotting using an anti-ubiquitin antibody.

MG-63 cells are grown in confluency in alpha MEM media and 10% fetal calf serum (FCS). Cells are then treated for 24 hours with specific compounds. Following the indicated treatments, cells are scraped with a disposable scraper, washed twice with phosphate saline solution (137 mM NaCl, 10 mM d-glucose, 4 mM KCI, 0.5 mM Na₂HPO₄, 0.1 mM KH₂PO₄), centrifuged, and the resulting pellet is suspended in the sample buffer containing 2% SDS, pH 6.75. The samples are heated and the concentration of total protein calculated by means of Micro bicinchoninic acid (BCA) Protein Assay Kit (Pierce, Rockford, IL/USA). The samples are diluted to obtain a final protein concentration of 2 mg/ml, supplemented with 10% 2-mercaptoethanol, 1% bromophenol blue and run on a 4-15% SDS-PAGE. Resulting gels are Western blotted with anti-ubiquitin rabbit polyclonal antibody (diluted 1:100; Sigma, St. Louis, MO/USA). The samples are visualized with horse-radish peroxidase coupled anti-rabbit IgG antibodies (Amersham Corp., Arlington Heights, IL/USA) using ECL detection kits (Amersham Corp.).

### NF-κB Activity Assays

Cells are treated with different concentrations of compounds, and nuclear extracts prepared. Briefly, cells are washed with phosphate-buffered saline, and resuspended in lysis buffer (0.6% Nonidet P-40, 150 mM NaCl, 10 mM Tris-HCl, pH 7.9, 1 mM EDTA, 0.5 mM DTT and a cocktail of protease inhibitors (Complete (TM), Boehringer Mannheim). After incubation on ice for 15 min, nuclei are collected by centrifugation. The pellet is resuspended in nuclear extraction buffer (10 mM Hepes, pH 7.9, 420 mM NaCl, 0.1 mM EDTA, 1.5 mM NgCl₂, 0.5 mM DTT, protease inhibitors (Complete (TM), Boehringer Mannheim), 25% glycerol), and incubated at 4 degrees C for 30 min. The supernatant is collected and dialyzed in a buffer containing 10 mM Tris0HCl, pH 7.5, 50 mM NaCl, 5 mM MgCl₂, 1 mM EDTA, 1 mM DTT, and 20% glycerol. After dialysis, the nuclear extract is centrifuged to remove precipitated proteins, and aliquots are stored at -70 C. Protein concentration in the nuclear extracts is measured by the method of Bradford using a dye-binding assay kit (Bio-Rad).

The probe for electrophoretic mobility shift assays is a 32P-labeled double-stranded oligonucleotide containing the consensus sequence specific for NF-κB (Promega). Nuclear extracts (5 ug) are pre-incubated in 20-ul reaction mixtures containing 10 mM Tris-HCl, pH 7.5, 50 mM NaCl, 2.5 mM DTT, 0.5 mM EDTA, 1 mM MgCl₂, 4% glycerol, and 5 ug of poly (dI-dC). After 10 min at room temperature, 10-20 frmol of probe is added, and incubated further for 20 min. DNA-protein complexes are separated from free oligonucleotides on a 5% polyacrylamide/0.5X TBE gel (45 mM Tris-HCl, 45 mM boric acid, 1 mM EDTA). After electrophoresis, gels are dried and autoradiographed.

### Assays for Production Inhibition

Compounds which inhibit the production of the enzymes having proteasomal activity or of NF-κB can be assessed by measuring the level of production of these proteins in the presence and absence of candidate compounds. The levels of production can be readily measured in *in vitro* systems using, for example, immunoassays for the level of protein produced. The levels of such proteins can also be assessed by utilizing, for example, methionine labeling and size separation of proteins in the cells to be assessed. In order to effect a convenient level of protein production for measurement, it is advantageous to use recombinant expression systems for the relevant enzymes or the NF-κB so that substantial amounts are produced.

Typical approaches to inhibiting the production of NF-κB or proteasome enzymes include the use of antisense technology or formation of triplexes with double-stranded forms of nucleotide sequences relevant in the expression of the genes. In addition, various small molecules may also inhibit this production.

### Assays - Hair Growth: In Vivo Assay of Effects of Compounds on Hair Follicles Proliferation and Hair Growth

The assay described above to assess the effect of compounds on calvarial bone growth can also be used to assess the ability of compounds to stimulate hair growth. The test compound or appropriate control vehicle is applied to the upper and lower back of male ICR Swiss white mice either topically or by subcutaneous injection. The vehicle is selected as appropriate for the compound to be tested and for the route of administration. Optionally, the hair in the test area may be removed prior to administration. After a suitable interval, typically 7 days, the mice are anesthetized and a biopsy of the dorsal treatment area is taken using a 6 mm dermal punch. The specimens are fixed in 10% buffered formalin and imbedded in paraffin wax, and sectioned and stained to observe hair follicles. In addition, photography can be used to observe and record hair growth; typically such growth is observed after 14-18 days. After a suitable interval, typically 21 days, the animals may be euthenized and the hair analyzed for fiber analysis and the tissue from the treatment area analyzed for quantitation of hair follicles.

### Nature of the Compounds Useful in the Invention

The compounds useful in the methods and uses of the invention are inhibitors of proteasomal activity and preferably also inhibitors of the transcription factor NF-κB. Known inhibitors of these activities can be ascertained from the literature or compounds can be tested for these activities using assays known in the art. In addition, inhibitors which lower the level of effective expression of the nucleotide sequence encoding the enzymes that have proteasomal activity can be assessed and used in the invention methods.

The compounds thus identified, include compounds that inhibit the isoprenoid pathway, such as the statins. A description of these compounds can be found in WO98/25460 and in U.S. Serial No. 09/096,631. For convenience, the isoprenoid pathway referred to is set forth herein in Figure 2. One class of compounds which are inhibitors are the statins which have the formula 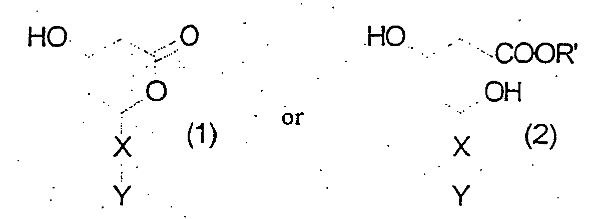
wherein X in each of formulas (1) and (2) represents a substituted or unsubstituted alkylene, alkenylene, or alkynylene linker of 2-6C;
Y represents one or more carbocyclic or heterocyclic rings wherein, when Y comprises two or more rings, said rings may be fused; and
R' represents a cation, H or a substituted or unsubstituted alkyl group of 1-6C; and the dotted lines represent optional π-bonds.

These compounds may, be used in the method of the invention as it relates to the stimulation of hair growth.

Compounds known to be proteasome or NF-κB inhibitors include:

| **Proteasome Inhibitors** | |
|---|---|
| PSI | N-carbobenzoyl-Ile-Glu-(OtBu)-Ala-Leu-CHO |
| MG-132 | N-carbobenzoyl-Leu-Leu-Leu-CHO |
| MG-115 | N-carbobenzoyl-Leu-Leu-Nva-CHO |
| MG-101 or Calpain Inh I | N-Acetyl-Leu-Leu-norLeu-CHO |
| ALLM | N-Acetyl-Leu-Leu-Met-CHO |
| | N-carbobenzoyl-Gly-Pro-Phe-Leu-CHO |
| | N-carbobenzoyl-Gly-Pro-Ala-Phe-CHO |
| | N-carbobenzoyl-Leu-Leu-Phe-CHO |
| | N-carbobenzoyl-Leu-Ala-Leu-CHO |
| Gliotoxin | 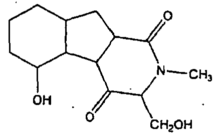 |
| SN50 | NLS of NF-κB MW 2781 |
| Bay 11-7082 | 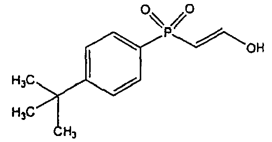 |
| Capsaicin | 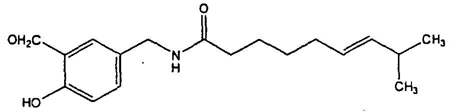 |
| PDTC | 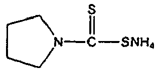 |

See, for example, Vinitsky, A*. et al. J Biol Chem* (1994) 269:29860-29866; Figueiredo-Pereira, M.E*. et al. JNeurochem* (1994) 63:1578-1581; Wojcik, C. *et al. Eur J Cell Biol* (1996) 71:311-318.

In the foregoing list, lactacystin is known to be an irreversible inhibitor of proteasome activity. It binds to the β catalytic subunit and is a specific inhibitor of the 20S proteasome. It also irreversibly inhibits NF-κB.

SN50 is the NLS (nuclear localization sequence) of p50 plus the hydrophobic region of K-FGF. It inhibits the translocation of the NF-κB active complex to the nucleus.

Certain peptidyl epoxy ketones such as EST are irreversible inhibitors of the proteasomes. MG-132 shows activity against the chymotryptic activity of the 20S protein without affecting its ATPase or isopeptidase activity and reversibly inhibits NF-κB activity. MG-115 and MG-341 show similar activities to MG-132. Various other inhibitors of NF-κB are less active in the ABA assay. These include capsaicin, curcumin, and resiniferatoxin. Other compounds known to inhibit NF-κB are gliotoxin and PDTC (1-pyrrolidine carbothiotic acid). Various other compounds such as BAY-11-7082 and BAY-11-7085 as well as calyculin-A inhibit phosphorylation of NF-κB. Calpain inhibitor inhibits calpain 1 and the proteasome; other compounds such as olomoucine and roscovitine inhibit cdk2 and/or cdk5.

An additional compound shown to be a proteasome inhibitor is pentoxyfilline (PTX). Combaret. L. et al. Mol Biol Rep (1999) 26:95-101. It is active in the in vitro calvarial assay described above.

As set forth above, in preferred embodiments of the methods of the invention, the identified compounds inhibit the isoprenoid pathway, typically as shown in Figure 1. In other preferred embodiments, compounds are described in PCT applications WO98/17267, WO97/15308, and WO97/48694 cited. The use of these compounds in the method to stimulate hair growth according to the invention is included.

The following examples are intended to illustrate but not to limit the invention.

### Example 1

### High Throughput Screening

Thousands of compounds have been tested in the assay system set forth in U.S. Serial No. 08/458,434, filed 2 June 1995. Representative compounds of the invention gave positive responses, while the majority of (unrelated) compounds are inactive. In this screen, the standard positive control was the compound 59-0008 (also denoted "OS8"), which is of the formula:

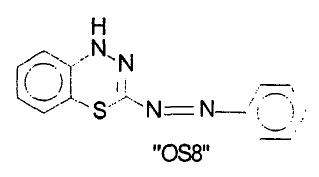

In more detail, the 2T3-BMP-2-LUC cells, a stably transformed osteoblast cell line described in Ghosh-Choudhury *et al. Endocrinology* (1996) 137:331-39, referenced above, was employed. The cells were cultured using α-MEM, 10% FCS with 1% penicillin/streptomycin and 1% glutamine ("plating medium"), and were split 1:5 once per week. For the assay, the cells were resuspended in a plating medium containing 4% FCS, plated in microtiter plates at a concentration of 5 x 10³ cells (in 50 µl)/well, and incubated for 24 hours at 37°C in 5% CO₂. To initiate the assay, 50 µl of the test compound or the control in DMSO was added at 2X concentration to each well, so that the final volume was 100 µl. The final serum concentration was 2% FCS, and the final DMSO concentration was 1%. Compound 59-0008 (10 µM) was used as a positive control.

The treated cells were incubated for 24 hours at 37°C and 5% CO₂. The medium was then removed, and the cells were rinsed three times with PBS. After removal of excess PBS, 25 µl of 1X cell culture lysing reagent (Promega #E153A) was added to each well and incubated for at least ten minutes. Optionally, the plates/samples could be frozen at this point. To each well was added 50 µl of luciferase substrate (Promega #E152A; 10 ml Promega luciferase assay buffer per 7 mg Promega luciferase assay substrate). Luminescence was measured on an automated 96-well luminometer, and was expressed as either picograms of luciferase activity per well or as picograms of luciferase activity per microgram of protein.

In this assay, compound 59-0008 (3-phenylazo-1H-4,1,2-benzothiadiazine) exhibits a pattern of reactivity which is maximal at a concentration of approximately 3-10 µM. Accordingly, other tested compounds can be evaluated at various concentrations, and the results compared to the results obtained for 59-0008 at 10 µM (which value would be normalized to 100). Alternatively, the reactivity of a compound to be tested can be compared directly to a negative control containing no compound.

The control compound 59-0328, which is simvastatin, gives a good response. The known proteasome inhibitors MG-132 and MG-115 also show high activity; MG-132 is effective at lower concentrations. Positive responses are also obtained using lactacystin. However, gliotoxin, olomoucine, roscovitine, SN50, PDTC, and capsaicin do not give promising responses.

### Example 2

### In vitro Bone Formation

Selected compounds and appropriate controls were assayed *in vitro (ex vivo*) for bone formation activity (described above in "Techniques for Neonatal Mouse Calvaria Assay *(in vitro)).* Histomorphometrical assessments of *ex vivo* calvaria were carried out using an OsteoMetrics bone morphometry measurement program, according to the manufacturer's instructions. Measurements were determined using either a 10- or 20-fold objective with a standard point counting eyepiece graticule.

New bone formation was determined (using a 10X objective) by measuring the new bone area formed in one field in 3 representative sections of each bone (4 bones per group). Each measurement was carried out ½ field distance from the end of the suture. Both total bone and old bone area were measured. Data were expressed as new bone area in µm².

The results in Example 1 were somewhat imperfectly correlated with the results in this assay. The control compound, simvastatin showed new bone formation in this assay as did MG-132 and lactacystin. MG-115 also showed positive results although less dramatic than those of simvastatin. However, gliotoxin, which appeared negative in the ABA assay of Example 1 did demonstrate the ability to stimulate bone growth. The remaining compounds, olomoucine, roscovitine, SN50, PDTC and capsaicin appeared negative in this assay.

Osteoblast numbers are determined by point counting. The number of osteoblast cells lining the bone surface on both sides of the bone are counted in one field using a 20X objective. Data are expressed as osteoblast numbers/mm of bone surface.

Alkaline phosphatase activity is measured in the conditioned media of the murine organ cultures, using the method described by Majeska, R.J. *et al. Exp Cell Res* (1978) 111:465-465. Conditioned media are incubated at 37°C for 20 minutes with phosphatase substrate 104 (Sigma) and the reaction stopped with 2 ml of 0.1 M NaOH. Alkaline phosphatase activity is calculated by measuring cleaved substrate at an optical density of 410 nm in a Beckman dual beam spectrophotometer from the OD410 and corrected for protein concentration.

PSI and MG-132 and control compounds/factors bFGF and BMP-2, and a vehicle control were tested in this assay and the calvaria were analyzed histomorphometrically, as described above. Increase in bone area as a function of concentration; the increase in osteoblasts and the enhancement of alkaline phosphatase activity for PSI were measured.

The data show that PSI is as good as, or better than, BMP-2 and bFGF (two "gold standard" agents for bone growth; see Wozney J. *Molec Reprod Dev* (1992) 32:160-67; WO95/24211) for inducing bone formation.

An additional experiment, pentoxyfilline (PTX) was tested in the foregoing assay. It exhibited the ability to enhance new bone formation in concentrations as low as 0.1 µm. At a concentration of 10 µm, PTX appeared to enhance the new bone are over control by over 100%; at 100 µm, the increase was approximately three (3) times that of control.

### Example 3

### In vivo Calvarial Bone growth Data

PSI and MG-132 were assayed *in vivo* according to the procedure described previously (see *"In vivo* Assay of Effects of Compounds on Murine Calvarial Bone Growth", *supra).* As a control, simvastatin provided a 1.5 fold increase in the number of osteoblasts.

In one experiment, vehicle control, bFGF and varying doses of PSI were tested in the *in vivo* calvarial bone growth assay. The results are reported as a measurement of total bone area, % increase in area over vehicle control, and % increase in new bone width as shown below.

| **Compound** | **Total Bone Area (µm**^{**2**}**)** | **% Increase* in Bone Area Compared with Control** | **% Increase**^{**+**} **in New Bone Width** |
|---|---|---|---|
| Control | 0.64 ± 0.03 | | |
| 0.1 mg/kg/day | 0.74 ± 0.02 | 21.7 ± 3.5 | |
| 1 mg/kg/day | 0.83 ± 0.02 | 35.4 ± 3.4 | 19.9 ± 2.0 |
| 5 mg/kg/day | 0.79 ± 0.03 | 32.1 ± 5.6 | 19.9 ± 4.4 |
| | | ^{*}p < 0.05 | ⁺p < 0.001 |

In addition, histological examination showed confirmation of bone growth both when 5 mg/kg/day of PSI was used and 1 mg/kg/day was used.

### Example 4

### Summary of Effects on Bone Formation

The table below summarizes the results obtained for compounds tested in the various assays set forth above. It is seen that compounds that are proteasome inhibitors also enhance bone formation. In the compounds tested in this table, however, compounds which are known to be inhibitors only of NF-κB but which fail to inhibit proteasomal activity, do not enhance luciferase activity (indicative of BMP-2 promoter activity) in the high through-put assay, nor do they enhance bone formation in the calvarial assay *in vitro,* to as great an extent as do proteasome inhibitors.

Compounds useful in the invention include:

| **Compound** | **Structure** | | **Luciferase Activity (ED**_{**50**}**-µM)** ⇑ | | **Bone Formation (ED**_{**50**}**-µM)** | | **Proteasome Activity (ED**_{**50**}**-µM)** |
|---|---|---|---|---|---|---|---|
| Simvastatin | 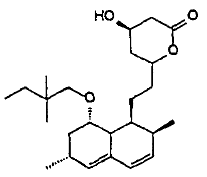 | ⇑ | 0.2 | ⇓ | 0.2 | ⇓ | - |
| Lactacystin | 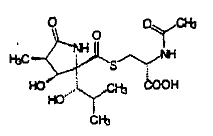 | ⇑ | 1 | ⇓ | 1 | ⇓ | 1.5 |
| PS1 | Z-lie-Glu(OtBu)-Ala-Leu-CHO | ⇑ | 0.05 | ⇓ | 0.03 | ⇓ | 0.035 |
| MG132 | 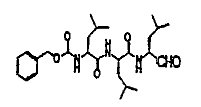 Z-Leu-Leu-Leu-CHO | ⇑ | 0.25 | ⇓ | 0.5 | ⇓ | 0.3 |
| MG262 | 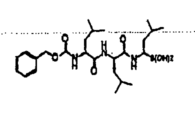 | ⇑ | 0.1 | ⇓ | 0.1 | ⇓ | 0.07 |
| MG115 | 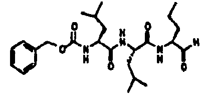 Z-Leu-Leu-Nva-CHO | ⇑ | 2 | ⇓ | 1 | ⇓ | 1 |
| ALLN | 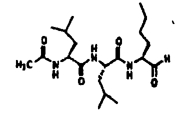 N-Acetyl-Leu-Leu-Nle-CHO | ⇑ | 10 | | - | ⇓ | 1.5 |
| Cyclosporin A | 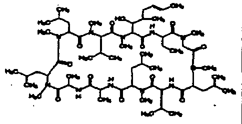 | - | - | ⇑ | 10 | ⇓ | 1.0 |
| Gliotoxin | 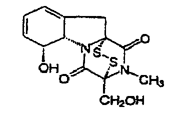 | - | - | ⇑ | 10 | - | - |
| SN50 | NLS of NF-KB MW 2781 | - | - | - | - | - | - |
| ALLM | N-Acetyl-Leu-Leu-Meth-CHO | - | - | - | - | ⇓ | 4 |
| PPM-18 | 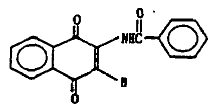 | - | - | - | - | - | - |
| Bay 11-7082 | 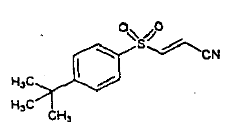 | - | - | | - | | - |
| Capsaicin | 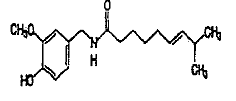 | - | - | | - | ⇓ | 30 |
| PDTC | 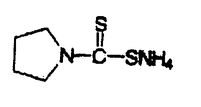 | - | - | - | - | - | - |

### Example 5

### Effect of PSI and Other Proteasome Inhibitors on Hair Follicle Production

The *in vivo* bone calvarial growth assay of Example 3 was modified to observe the number of hair follicles in treated mice. In initial observations, PSI (5mg/kg/day) was injected three times a day for 5 days over the calvaria of Swiss ICR mice as described above. Sixteen days later the mice were sacrificed. Histology of the calvaria revealed a strikingly large increase in the number of hair follicles in those mice treated with PSI versus control mice. In addition to PSI, MG132 (10mg/kg), MG115 (10mg/kg) and lactacysin administered in the same way also stimulated an increase in the number of hair follicles.

### Example 6

### Stimulation of Hair Growth

Male Swiss ICR mice were first treated to remove hair from the scalp and dorsal regions as follows. Paraffin wax was liquefied by heating to 55°C and the liquefied wax then applied by brush to the scalp and/or back (under anesthesia). The wax was allowed to solidify and then removed. The day following hair stripping, PSI (1 mg/kg/day) was injected subcutaneously three times a day for five days into the scalp and dorsal region. On day 7 a dermal punch biopsy was taken; histology revealed a large increase in the number of hair follicles in mice administered PSI versus control mice. By day 18, it was observable that the treated mice had a hair growth rate greater than that of the mice in the control group.

The mice were sacrificed on day 21 and histology was performed on the dermis of the scalp and of the dorsal region. In the treated mice, mature hair follicles in numbers much greater than in controls had migrated to the lower region of the dermis. Upon closer examination, it was observed that mice that had received only vehicle had quiescent hair follicles. When treated with PSI such follicles were stimulated to differentiate into mature hair follicles and to migrate to the lower region of the dermis.

### Example 7

### Topical Administration

PSI was prepared as a topical formulation, where the vehicle was 50% propylene glycol, 30% ethanol, 20% deionized water, at 0.1 % concentration of PSI. The solution was applied 3 times a day for 5 days. The mice in a treated group were observed as compared to controls similarly treated with vehicle alone. The results at day 16 showed stimulation of hair growth relative to the controls.

In addition to stimulating hair growth, PSI was able to thicken both the hair and the hair shaft. PSI increases hair count when the follicle area is greater than 0.01 mm². When the protocol above was repeated using a 0.5% solution of PSI in groups containing 5 mice each, the number of hairs per 0.8 mm² was 60 in the treated mice versus about 10 in the control group. The percentage of follicle area in a region of about 0.8 mm² was about 30% as an average in the treated group as compared to 15% as an average in the control group.

### Example 8

### Dose Requirements

In order to determine the minimal effective dosage of PSI, when used topically, a dose response curve for PSI was prepared. All experiments were preformed according to current good laboratory practice regulations (21CFR58). The mice were divided into 7 groups, 10 mice each, wherein one group was control treated only with vehicle and groups 1-6 with a series of increasing concentrations of PSI in a vehicle comprising 50% propylene glycol, 30% ethanol, 20% deionized water. The concentrations were 0.006%, 0.012%, 0.025%, 0.05%, 0.11% and 0.5%.

The mice were anesthetized (50 µl Mouse Cocktail containing 3 ml ketamine, 2 ml small animal rompum, 5 ml NaCl), identified by ear punch code, weighed and the hair on the dorsal side removed by waxing as described in Example 6. After waxing, the animals were photographed. On the following day (day 1), 100 µl of PSI at the above concentrations in vehicle was brushed onto the area of removed hair. A similar application of PSI solution was performed daily for an additional 4 days.

On Day 7 mice were anesthetized and a biopsy of the dorsal treatment area taken using a 6 mm dermal punch; the specimens were fixed in 10% buffered formalin and embedded in paraffin wax.. Sections were cut using a standard microtome.

Mice were monitored daily for signs of hair growth, and any hair growth was recorded by photography. On day 21 animals were euthanized (75mg/kg body weight phenobarbital, IP injection), 2 cm hair samples were taken for optical based fiber analysis, and the remaining dorsal treatment area was fixed in 10% buffered formalin for further histological analysis. Analysis included quantification of hair thickness and quantification of mature hair follicles. Results were expressed as the mean = +/- the standard error of the mean. Data were analyzed by repeated measures of analysis of variance followed by the Tukey-Kramer post test P values of <0.05 were considered significant.

The results indicate that the minimal effective dose of PSI is 0.5% applied 1 time a day for 4 days; additional experiments showed that 0.1 % of PSI applied topically 3 times a day for 5 days was also effective.

Gross observation of mice receiving an effective dose indicated an enhanced rate of hair growth, a thickening of hair diameter, increase in sheath diameter, and differentiation of quiescent hair follicles into more mature forms.

## Claims

1. A cosmetic method to treat a mammalian subject for a condition benefited by stimulating hair growth which method comprises administering to said mammalian subject an amount effective to stimulate hair growth of a compound that inhibits proteasomal activity or that inhibits production of these proteins, wherein said compound is a statin, a peptidyl aldehyde or a peptidyl epoxyketone, or is selected from the group consisting of pentoxyfilline, gliotoxin, SN50, Bay 11-7082, capsaicin, PDTC, PPM-18, lactacystin and MG262.

2. The method of claim 1, wherein said compound is a statin or is PSI.

3. The method of claim 1 or 2, wherein said subject is human.

4. The method of claim 1, wherein said condition to be treated is male pattern baldness, alopecia caused by chemotherapy, hair thinning due to aging, or a genetic disorder.

5. The method of claim 1 or 2, wherein said subject is a non-human mammal.

6. The method of claim 5, wherein said hair growth provides additional protection from cold temperatures.

7. The method of any one of claims 1 to 6, wherein said hair growth is **characterised by** increased rate of growth in hair length and/or thickness, or the appearance of proliferation of new hair follicles.

8. The method of any of claims 1 to 7, wherein said compound is co-administered in a biodegradable film or matrix with an agent promoting tissue growth or infiltration.

9. The method of claim 8, wherein said agent is an epidermal growth factor, a fibroblast growth factor, a platelet-derived growth factor, a transforming growth factor, a parathyroid hormone, a leukemia inhibitor/factor, or an insulin-like growth factor.

10. Use of a compound that inhibits proteasomal activity or that inhibits production of these proteins in the manufacture of a medicament for stimulating hair growth in a mammalian subject wherein said compound is a statin, a peptidyl aldehyde, or a peptidyl epoxyketone, or is selected from the group consisting of pentoxyfilline, gliotoxin, SN50, Bay 11-7082, capsaicin, PDTC, PPM-18, lactacystin and MG262.

11. The use of claim 10, wherein said compound is a statin or is PSI.

12. The use of claim 10 or 11, wherein the medicament is for the treatment of male pattern baldness, alopecia caused by chemotherapy, hair thinning due to aging, or a genetic disorder.

13. The use of any of claims 10 to 12, wherein said growth is **characterised by** increased rate of growth in hair length and/or thickness, or the appearance of proliferation of new hair follicles.

14. The use of any of claims 10 to 13, wherein said compound is co-administered in a biodegradable film or matrix with an agent promoting tissue growth or infiltration.

15. The use of claim 14, wherein said agent is an epidermal growth factor, a fibroblast growth factor, a platelet-derived growth factor, a transforming growth factor, a parathyroid hormone, a leukemia inhibitor/factor, or an insulin-like growth factor.

16. A method to identify a compound that stimulates hair growth which method comprises
subjecting a candidate compound to an assay to assess its ability to inhibit proteasomal activity, whereby a compound which inhibits proteasomal activity is identified as a compound that stimulates hair growth; or
subjecting a candidate compound to an assay to assess its ability to inhibit the production of enzymes with proteasomal activity, whereby a compound which inhibits the production of enzymes with proteasomal activity is identified as a compound that stimulates hair growth.

## Patentansprüche

1. Kosmetisches Verfahren zur Behandlung eines Zustands, bei dem die Stimulation von Haarwachstum von Vorteil ist, bei einem Säugetiersubjekt, wobei das Verfahren die Verabreichung einer zur Stimulation von Haarwachstum wirksamen Menge einer Verbindung, die die proteasomale Aktivität hemmt oder die Erzeugung dieser Proteine hemmt, an das Säugetiersubjekt umfasst, wobei es sich bei der Verbindung um ein Statin, einen Peptidylaldehyd oder ein Peptidylepoxyketon handelt oder die Verbindung aus der Gruppe, die aus Pentoxyfillin, Gliotoxin, SN50, Bay 11-7082, Capsaicin, PDTC, PPM-18, Lactacystin und MG262 besteht, ausgewählt ist.

2. Verfahren nach Anspruch 1, wobei es sich bei der Verbindung um ein Statin oder um PSI handelt.

3. Verfahren nach Anspruch 1 oder 2, wobei es sich bei dem Subjekt um einen Menschen handelt.

4. Verfahren nach Anspruch 1, wobei es sich bei dem zu behandelnden Zustand um das männliche Kahlköpfigkeitsmuster, durch Chemotherapie hervorgerufene Alopezie, Haarausdünnung aufgrund des Alterungsprozesses oder eine genetische Störung handelt.

5. Verfahren nach Anspruch 1 oder 2, wobei es sich bei dem Subjekt um ein nicht-humanes Säugetier handelt.

6. Verfahren nach Anspruch 5, wobei das Haarwachstum einen zusätzlichen Schutz gegen kalte Temperaturen gewährleistet.

7. Verfahren nach einem der Ansprüche 1 bis 6, wobei das Haarwachstum durch eine erhöhte Wachstumsrate der Haarlänge und/oder Haardicke oder durch das Auftreten der Proliferation von neuen Haarfollikeln **gekennzeichnet** ist.

8. Verfahren nach einem der Ansprüche 1 bis 7, wobei die Verbindung in einem biologisch abbaubaren Film oder Matrix zusammen mit einem Mittel, das das Gewebewachstum oder die Infiltration fördert, verabreicht wird.

9. Verfahren nach Anspruch 8, wobei es sich bei dem Mittel um einen epidermalen Wachstumsfaktor, einen Fibroblastenwachstumsfaktor, einen von Blutplättchen abgeleiteten Wachstumsfaktor, einen transformierenden Wachstumsfaktor, ein Parathyroidhormon, einen Leukämie-Inhibitor/Faktor oder einen insulinartigen Wachstumsfaktor handelt.

10. Verwendung einer Verbindung, die die proteasomale Aktivität hemmt oder die Bildung dieser Proteine hemmt, bei der Herstellung eines Arzneimittels zur Stimulation von Haarwachstum bei einem Säugetiersubjekt, wobei es sich bei der Verbindung um ein Statin, einen Peptidylaldehyd oder ein Peptidylepoxyketon handelt oder die Verbindung aus der Gruppe, die aus Pentoxyfillin, Gliotoxin, SN50, Bay 11-7082, Capsaicin, PDTC, PPM-18, Lactacystin und MG262 besteht, ausgewählt ist.

11. Verwendung nach Anspruch 10, wobei es sich bei der Verbindung um ein Statin oder um PSI handelt.

12. Verwendung nach Anspruch 10 oder 11, wobei das Arzneimittel zur Behandlung von männlichem Kahlköpfigkeitsmuster, durch Chemotherapie hervorgerufener Alopezie, Haarausdünnung aufgrund des Alterungsprozesses oder einer genetischen Störung dient.

13. Verwendung nach einem der Ansprüche 10 bis 12, wobei das Haarwachstum durch eine erhöhte Wachstumsrate der Haarlänge und/oder Haardicke oder durch das Auftreten der Proliferation von neuen Haarfollikeln **gekennzeichnet** ist.

14. Verwendung nach einem der Ansprüche 10 bis 13, wobei die Verbindung in einem biologisch abbaubaren Film oder Matrix zusammen mit einem Mittel, das das Gewebewachstum oder die Infiltration fördert, verabreicht wird.

15. Verwendung nach Anspruch 14, wobei es sich bei dem Mittel um einen epidermalen Wachstumsfaktor, einen Fibroblastenwachstumsfaktor, einen von Blutplättchen abgeleiteten Wachstumsfaktor, einen transformierenden Wachstumsfaktor, ein Parathyroidhormon, einen Leukämie-Inhibitor/Faktor oder einen insulinartigen Wachstumsfaktor handelt.

16. Verfahren zum Identifizieren einer Verbindung, die das Haarwachstum stimuliert, wobei das Verfahren folgendes umfasst:
eine Kandidatenverbindung wird einem Test zur Bestimmung ihrer Fähigkeit zur Hemmung der proteasomalen Aktivität unterworfen, wobei eine Verbindung, die die proteasomale Aktivität hemmt, als eine Verbindung identifiziert wird, die das Haarwachstum stimuliert; oder
eine Kandidatenverbindung wird einem Test zur Bestimmung ihrer Fähigkeit zur Hemmung der Erzeugung von Enzymen mit proteasomaler Aktivität unterworfen, wobei eine Verbindung, die die Erzeugung von Enzymen mit proteasomaler Aktivität hemmt, als eine Verbindung, die das Haarwachstum stimuliert, identifiziert wird.

## Revendications

1. Procédé cosmétique pour traiter un sujet mammifère, pour un état tirant avantage d'une stimulation de la croissance des poils, lequel procédé comprend l'administration, audit sujet mammifère, d'une quantité efficace pour stimuler la croissance des poils ou des cheveux, d'un composé qui inhibe l'activité protéasomique ou qui inhibe la production de ces protéines, ledit composé étant une statine, un peptidylaldéhyde ou une peptidylépoxycétone, ou étant choisi dans l'ensemble consistant en la pentoxyfilline, la gliotoxine, le SN50, le Bay 11-7082, la capsaicine, le PDTC, le PPM-18, la lactacystine et le MG262.

2. Procédé selon la revendication 1, dans lequel ledit composé est une statine ou un PSI.

3. Procédé selon la revendication 1 ou 2, dans lequel ledit sujet est un humain.

4. Procédé selon la revendication 1, dans lequel ledit état devant être traité est la calvitie hippocratique, l'alopécie provoquée par une chimiothérapie, l'amincissement des cheveux provoqué par le vieillissement, ou un trouble génétique.

5. Procédé selon la revendication 1 ou 2, dans lequel ledit sujet un mammifère non humain.

6. Procédé selon la revendication 5, dans lequel ladite croissance des poils assure une protection supplémentaire vis-à-vis des basses températures.

7. Procédé selon l'une quelconque des revendications 1 à 6, dans lequel ladite croissance des poils est **caractérisée par** une augmentation de la vitesse de croissance de la longueur et/ou de l'épaisseur des poils, ou par l'apparition d'une prolifération de nouveaux follicules pileux.

8. Procédé selon l'une quelconque des revendications 1 à 7, dans lequel ledit composé est co-administré dans un film ou une matrice biodégradable avec un agent favorisant la croissance ou l'infiltration tissulaire.

9. Procédé selon la revendication 8, dans lequel ledit agent est un facteur de croissance épidermique, un facteur de croissance des fibroblastes, un facteur de croissance dérivé des plaquettes, un facteur de croissance transformant, une hormone parathyroïdienne, un inhibiteur/ facteur de leucémie ou un facteur de croissance insulinomimétique.

10. Utilisation d'un composé qui inhibe l'activité protéasomique ou qui inhibe la production de ces protéines pour préparer un médicament destiné à stimuler la croissance des poils chez un sujet mammifère, ledit composé étant une statine, un peptidylaldéhyde, ou une peptidylépoxycétone, et étant choisi dans l'ensemble consistant en la pentoxyfilline, la gliotoxine, le SN50, le Bay 11-7082, la capsaicine, le PDTC, le PPM-18, la lactacystine et le MG262.

11. Utilisation selon la revendication 10, dans laquelle ledit composé est une statine ou un PSI.

12. Utilisation selon la revendication 10 ou 11, dans laquelle le médicament est destiné au traitement de la calvitie hippocratique, de l'alopécie provoquée par une chimiothérapie, de l'amincissement des cheveux provoqué par le vieillissement, ou d'un trouble génétique.

13. Utilisation selon l'une quelconque des revendications 10 à 12, dans laquelle ladite croissance est **caractérisée par** une augmentation de la vitesse de croissance de la longueur et/ou de l'épaisseur des poils, ou par l'apparition d'une prolifération de nouveaux follicules pileux.

14. Utilisation selon l'une quelconque des revendications 10 à 13, dans laquelle ledit composé est co-administré dans un film ou une matrice biodégradable avec un agent favorisant la croissance ou l'infiltration tissulaire.

15. Utilisation selon la revendication 14, dans laquelle ledit agent est un facteur de croissance épidermique, un facteur de croissance des fibroblastes, un facteur de croissance dérivé des plaquettes, un facteur de croissance transformant, une hormone parathyroïdienne, un inhibiteur/facteur de leucémie ou un facteur de croissance insulinomimétique.

16. Procédé d'identification d'un composé qui stimule la croissance des poils, lequel procédé consiste
à soumettre un composé candidat à un essai pour évaluer son aptitude à inhiber l'activité protéasomique, un composé qui inhibe l'activité protéasomique étant identifié comme étant un composé qui stimule la croissance des poils, ou bien
à soumettre un composé candidat à un essai pour évaluer son aptitude à inhiber la production d'enzymes ayant une activité protéasomique, un composé qui inhibe la production d'enzymes ayant une activité protéasomique étant identifié comme étant un composé qui stimule la croissance des poils.
